(19) Europäisches Patentamt

European Patent Office

Office européen des brevets

(11) **EP 0 955 543 B1**

(12) **FASCICULE DE BREVET EUROPEEN**

(45) Date de publication et mention
de la délivrance du brevet:
**16.04.2003  Bulletin 2003/16**

(51) Int Cl.⁷: **G01N 33/50**

(21) Numéro de dépôt: **99430007.7**

(22) Date de dépôt: **04.05.1999**

(54) **Nouveaux réactifs et méthode de lyse des érythrocytes**

Neue Reagenzien und Methoden zur Lyse von Erythrozyten

New reagents and methods for erythrocyte lysis

(84) Etats contractants désignés:
**AT BE CH DE DK ES FI FR GB GR IE IT LI LU MC
NL PT SE**

(30) Priorité: **07.05.1998  FR 9806055**

(43) Date de publication de la demande:
**10.11.1999  Bulletin 1999/45**

(73) Titulaire: **IMMUNOTECH S.A.
F-13276 Marseille Cédex 9 (FR)**

(72) Inventeurs:
• **van Agthoven, André
13009 Marseille (FR)**
• **Jarrossay, David
13006 Marseille (FR)**

(74) Mandataire: **Rinuy, Santarelli
14, avenue de la Grande Armée
75017 Paris (FR)**

(56) Documents cités:
**EP-A- 0 872 734          US-A- 3 884 579
US-A- 5 316 951          US-A- 5 763 280**

**Description**

La présente invention concerne de nouveaux réactifs et une méthode de lyse des érythrocytes sans toxicité pour les leucocytes et compatible avec l'utilisation d'un fixateur du type aldéhyde aliphatique.

Dans un sang normal, les érythrocytes sont environ mille fois plus abondants que les leucocytes et forment un obstacle pour toute étude ou analyse leucocytaire. C'est pourquoi des techniques d'analyse leucocytaires telles cytométrie, ou cytospin mettant en jeu la centrifugation des composants sanguins, sont précédées dans la plupart des cas par une étape de séparation entre érythrocytes et leucocytes (US-A4 284 412, EP-A-0 022 670).

Une telle séparation par centrifugation utilisant le Ficoll pour isoler les lymphocytes et les granulocytes à partir du sang a été décrite par Boyem dans Scandinavian Journal of Clinical and Laboratory Investigation 1967-68, Suppl. 94-101.305.293 and Pertoff dans Journal of Immunological Methods : 1980, 33:221-229.

Ces méthodes sont relativement difficiles à mettre en oeuvre, car elles nécessitent plusieurs lavages et centrifugations, ce qui peut amener à des pertes de cellules et donc un dosage par défaut. Pourtant, ces méthodes sont actuellement considérées comme méthodes de référence car la morphologie et la viabilité des leucocytes sont conservées après la séparation.

La lyse de sang en vue d'une analyse leucocytaire implique la destruction des globules rouges jusqu'à l'état de débris. Les méthodes de lyse visent par contre à une conservation optimale de la morphologie des leucocytes afin de permettre une analyse leucocytaire cytométrique et de bien distinguer les leucocytes des débris érythrocytaires.

La lyse des érythrocytes se base sur le principe général de faire passer une quantité de matériel par la membrane à partir de l'extérieur de l'érythrocyte. Pendant ce passage, l'état de la membrane se dégrade, soit par le gonflement de la cellule, soit par la dissolution de la membrane dans le matériel de passage.

La conservation de la morphologie leucocytaire pendant la lyse est basée sur un environnement plus riche en protéines autour de sa membrane : à l'extérieur et à l'intérieur, des protéines transmembranaires, et à l'intérieur, un cytosquelette développé.

L'avantage de ce support de membrane leucocytaire est pleinement exploité par l'utilisation des fixateurs comme l'aldéhyde formique qui pénètre par la membrane et fixe les structures protéiques par cross-linking à l'extérieur et à l'intérieur.

Le matériel utilisé pour le passage des membranes érythrocytaires sont en général de petites molécules neutres, d'abord l'eau elle-même, utilisée dans la lyse hypotonique ou l'eau en présence de diéthylène glycol, aldéhyde formique et acide citrique comme dans Chang et al. (brevet US 4 902 613). Parfois, la fixation et la lyse hypotonique sont réalisées à des étapes différentes comme décrit par Quintana (WO-89/0509) et van Agthoven (EP-A-0625706).

Dans le cas des lyses isotoniques comme décrit dans EP-A-625 707, le matériel de passage est constitué d'un mélange d'aldéhyde formique, de glycérol, de butanol et d'acide citrique.

Une autre technique de lyse isotonique utilise la saponine, une petite molécule aux propriétés détergentes comme décrit dans WO 85/05640.

Il existe une technique de lyse dans laquelle le principe général de la lyse n'est pas tout de suite apparent. Il s'agit de la lyse au chlorure d'ammonium. Le fonctionnement de la lyse dépend du passage de $NH_3$ et $CO_2$ par la membrane. $NH_3$ et $CO_2$ se trouvent en équilibre avec $NH_4^+$ et $HCO_3^-$ dans le mélange. La reconversion spontanée de $NH_3$ en $NH_4^+$ dans la cellule et de $CO_2$ en $HCO_3^-$ par l'anhydrase carbonique présente en grandes quantités dans l'érythrocyte pourrait être la force derrière un flux continu de $NH_3$ et $CO_2$ entrant dans la cellule.

La lyse au chlorure d'ammonium est la lyse la plus efficace qui puisse être effectuée en l'absence de fixateur et c'est en conséquence la méthode préférée de beaucoup de laboratoires de recherche. Une limitation de cette méthode est que les leucocytes perdent très vite leur viabilité pendant le processus de lyse. Apparemment, dans un premier temps, l'intérieur de la cellule est alcalinisé par le produit des deux réactions $NH_5CO_3$. Plus tard, dans la réaction après lyse et libération de l'anhydrase carbonique des érythrocytes, la réaction $HCO_3 \Rightarrow CO_2 + OH^-$ à partir du bicarbonate présent dans le tampon de lyse fait alcaliniser le mélange total, ce qui provoque la dégradation des leucocytes. A cause de ces conditions toxiques, la lecture doit être effectuée rapidement, habituellement 1 à 2 heures après la réalisation de la préparation.

Une autre limitation de la méthode est l'impossibilité de combiner l'utilisation de chlorure d'ammonium avec l'aldéhyde formique en raison de la réaction suivante :

$$6HCHO + 4NH_3 \rightarrow C_6H_{12}N_4 + 6H_2O.$$

L'hexaméthylènetétramine formée est stable et en conséquence le mélange s'acidifie pendant la réaction au détriment des leucocytes parce que l'élimination de $NH_3$ dans le milieu déplace l'équilibre et les ions $H^+$ ne sont plus compensés.

En résumé le chlorure d'ammonium s'est avéré être incompatible avec les aldéhydes aliphatiques et de ce fait la lyse s'arrête rapidement en cas d'utilisation conjointe.

Il serait donc souhaitable de disposer d'un réactif de lyse et d'une méthode de lyse des érythrocytes conservant au maximum la viabilité cellulaire des leucocytes. Il serait aussi souhaitable de disposer d'un réactif de lyse et d'une méthode de lyse des érythrocytes permettant d'effectuer la lecture cytométrique longtemps après la réalisation de la préparation, par exemple un jour et plus après la mise en réaction des produits.

Il serait tout aussi souhaitable de disposer d'un réactif de lyse et d'une méthode de lyse des érythrocytes compatibles avec l'utilisation d'aldéhyde formique.

Or après de longues recherches, la demanderesse a découvert que les problèmes ci-dessus étaient résolus par l'utilisation à titre d'agent de lyse d'un hétérocycle azoté.

C'est pourquoi la présente demande a pour objet un réactif de lyse utilisable pour l'analyse cytométrique des leucocytes, caractérisé en ce que l'agent de lyse est un hétérocycle azoté utilisé à un pH compris entre 4 et 9, de préférence compris entre 5 et 8, notamment compris entre 6 et 7,5, tout particulièrement compris entre 6,8 et 7,2.

Par « agent de lyse », l'on entend que l'hétérocycle azoté est le principal agent de lyse employé.

La demanderesse a en effet découvert que l'utilisation, à titre d'agent de lyse, d'un hétérocycle azoté donnait des résultats comparables à $NH_4Cl$ tout en étant compatible avec l'utilisation d'aldéhyde formique pour protéger les leucocytes. Il semblerait que les aldéhydes aliphatiques réagissent avec les hétérocycles azotés, mais la réaction dans le milieu de lyse n'est pas totale et les produits restent en général en équilibre sans former un produit de réaction stable.

L'hétérocycle azoté peut être par exemple bicylique et de préférence monocyclique. Il peut être insaturé et dans ce cas comporte par exemple 5, de préférence 4, notamment 3, particulièrement 2 doubles liaisons, et il est de préférence saturé. Il comprend par exemple de 3 à 8, notamment de 3 à 6 et particulièrement de 3 à 5, et tout particulièrement 4 ou 5 atomes de carbone. Il comprend 2, notamment 1 seul atome d'azote.

On peut citer par exemple à titre d'hétérocycle azoté saturé la pyrazolidine, l'imidazolidine et l'imidazoline, la pipérazine, notamment la morpholine et particulièrement la pipéridine ou la pyrrolidine.

Dans un réactif de lyse selon l'invention, l'hétérocycle azoté peut être présent à la concentration molaire de 0,01 à 0,2M, particulièrement de 0,05 à 0,19M et tout particulièrement de 0,1 à 0,18M. Dans des conditions tout à fait préférentielles de mise en oeuvre du réactif de lyse décrit ci-dessus, on utilise une concentration de 0,15M.

Les concentrations sont données en relation avec la quantité du composé concerné dans le milieu pendant la lyse érythrocytaire.

Dans d'autres conditions préférentielles de mise en oeuvre de l'invention, le composé utilisé selon l'invention pour conférer le pH désiré au réactif de lyse décrit ci-dessus utilise le chlorure comme anion, par exemple sous forme de HCl. Ce composé est de préférence un acide.

Dans toujours d'autres conditions préférentielles de mise en oeuvre, le réactif de lyse de l'invention comprend en outre, en dehors de l'anion utilisé selon l'invention pour conférer le pH désiré au réactif de lyse décrit ci-dessus, un contre-ion qui est un carbonate ou hydrogénocarbonate mais de préférence un borate. Ces ions peuvent être apportés sous forme d'acide mais de préférence sous forme de sel par exemple de sodium, particulièrement de potassium.

En effet, il a été trouvé que le borate n'était pas soumis à une dégradation comme le bicarbonate et contribue donc à une bonne stabilisation des cellules permettant une lecture cytométrique longtemps après la réalisation de la lyse. De plus, son utilisation permet de préparer une solution de lyse stable, ce qui n'est pas le cas avec l'hydrogénocarbonate.

Dans un réactif de lyse selon l'invention, le contre-ion peut être présent à la concentration molaire de 0,001 à 0,2M, particulièrement de 0,005 à 0,1M et tout particulièrement de 0,01 à 0,05M. Selon l'invention, le borate est utilisé dans des concentrations plus élevées que le carbonate. On utilise par exemple une concentration de 0,04M borate pour une concentration de 0,01M de carbonate ou hydrogénocarbonate.

Dans d'autres conditions préférentielles de mise en oeuvre de l'invention, le réactif de lyse de l'invention comprend en outre une quantité efficace d'un agent tampon particulièrement aux pH 6,5 à 7,5 . On peut citer par exemple à titre d'agent tampon le MES (Acide 2-(N-morpholino)éthane sulfonique), notamment le MOPS (Acide 3-(N-morpholino)propane sulfonique) et particulièrement l'HEPES (Acide N-(2-hydroxyéthyl)pipérazine-N'-(2-éthane sulfonique)).

Dans un réactif de lyse selon l'invention, l'agent tampon peut être présent à la concentration molaire de 0,0001 à 0,05M, particulièrement de 0,0005 à 0,03M et tout particulièrement de 0,002 à 0,008M.

Dans encore d'autres conditions préférentielles de mise en oeuvre de l'invention, le réactif de lyse de l'invention comprend en outre une quantité efficace d'un agent anticoagulant.

On peut citer par exemple à titre d'agent anti-coagulant l'héparine, notamment l'ion citrate, et particulièrement l'EDTA.

Dans des conditions tout à fait préférentielles de mise en oeuvre de l'invention, on utilise un mélange de 0,15M chlorhydrate de pyrrolidine, 0,04M d'acide borique, 0,0001 M d'ETDA et 0,005M d'HEPES à un pH autour de 7,0.

Dans encore d'autres conditions préférentielles de mise en oeuvre du réactif de lyse ci-dessus décrit, on utilise de surcroît un agent de fixation, notamment un aldéhyde aliphatique tel qu'en $C_1$-$C_5$, par exemple le paraformaldéhyde et particulièrement l'aldéhyde formique.

L'aldéhyde aliphatique peut être présent dans une concentration de 0,01% à 5 %, particulièrement de 0,14% à 1% et tout particulièrement de 0,1% à 0,5%. Dans des conditions préférentielles de mise en oeuvre du réactif de lyse ci-dessus décrit avec fixateur, on utilise 0,15M de chlorhydrate de pyrrolidine, 0,04M d'acide borique, 0,0001M d'EDTA, 0,005M d'HEPES et 0,3% d'aldéhyde formique.

La présente demande a encore pour objet une méthode de lyse des érythrocytes dans laquelle on soumet à l'action d'un réactif de lyse ci-dessus décrit un échantillon de sang total traité avec un agent d'anti-coagulation comme l'EDTA, l'héparine ou les ions citrate.

On peut opérer en l'absence ou en présence d'anticorps monoclonaux pour effectuer le marquage des cellules leucocytaires. Ces anticorps peuvent ou non être liés à un composé fluorescent tel que ceux décrits ci-après. Dans des conditions préférentielles de mise en oeuvre, ces anticorps sont liés à un composé fluorescent.

Les réactifs de lyse ci-dessus décrits peuvent être utilisés comme suit:

Un échantillon de 0,1 ml de sang traité avec un agent d'anti-coagulation, et incubé préalablement avec un anticorps monoclonal ou un anticorps monoclonal conjugué à un marqueur de fluorescence, ou un mélange d'anticorps monoclonaux conjugués à des marqueurs de fluorescence est mis en contact avec 2 ml de réactif de lyse ci-dessus préchauffé à une température de 37°C et laissé à refroidir pendant dix minutes au cours desquelles la lyse se termine. De tels marqueurs sont par exemple le CD45-FITC (CD45 conjugué à l'isothiocyanate de fluorescéïne) ou le CD14 conjugué à la phycoérythrine et sont commercialisés par exemple par les sociétés DAKO, BECTON et DICKINSON ou IMMUNOTECH. On procède alors à la lecture dans un cytomètre par exemple de type BECTON et DICKINSON Facscan, soit immédiatement, soit jusqu'à 3 jours après la lyse.

Dans des conditions préférentielles de mise en oeuvre de l'utilisation selon l'invention, on choisit les conditions préférentielles ci-dessus décrites pour le réactif et la méthode de lyse.

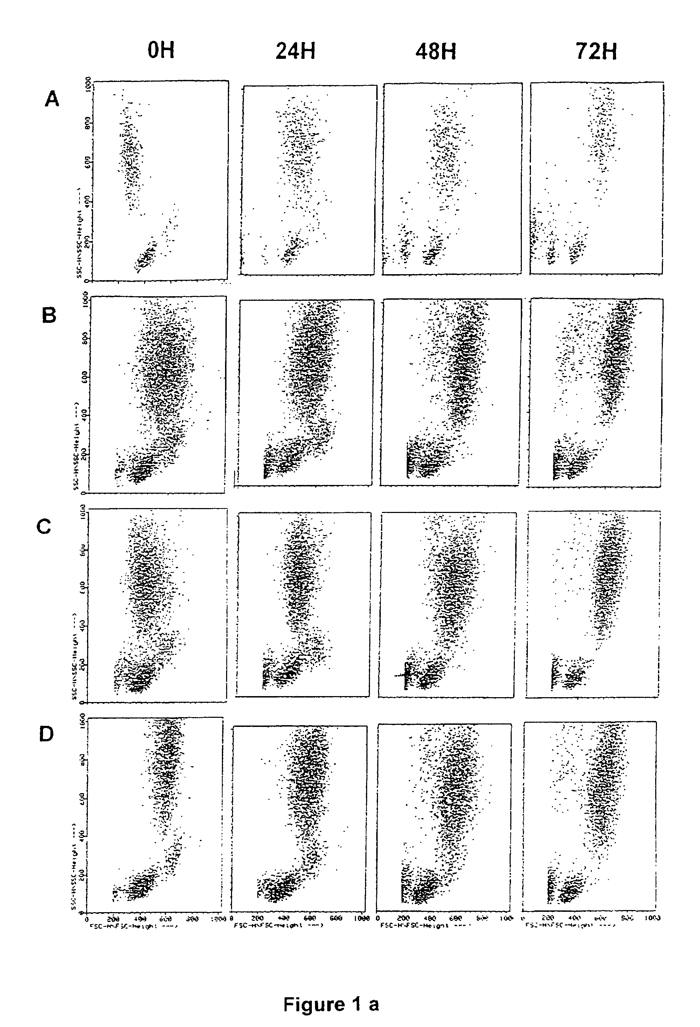

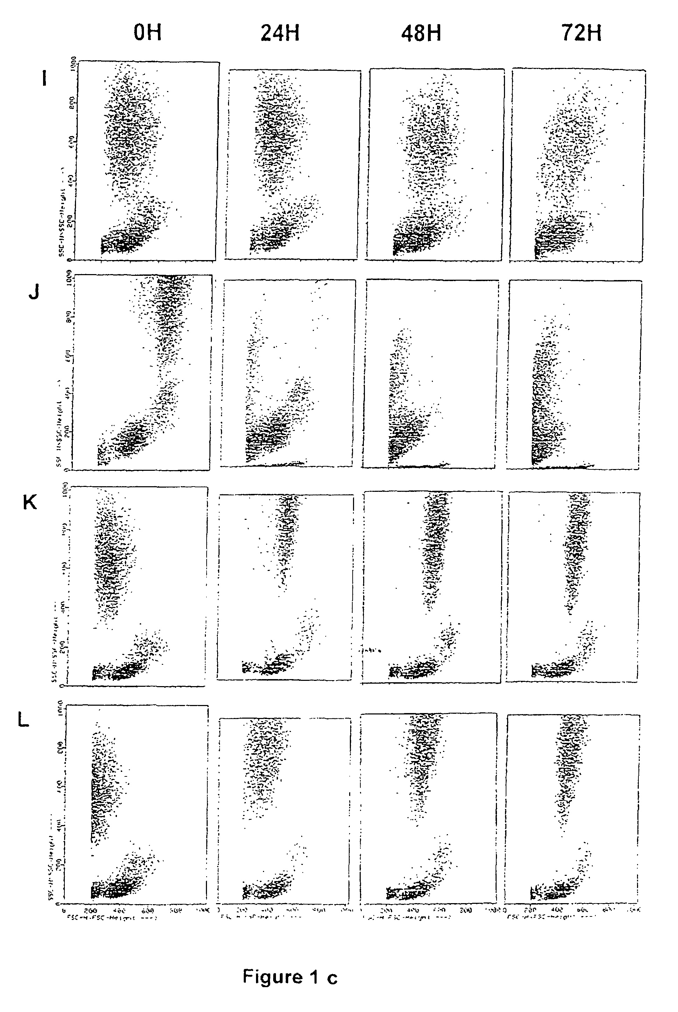

Les figures 1 A à 1 M, représentent les résultats obtenus par cytométrie en utilisant un Facscan de BECTON et DICKINSON en comparant la diffusion aux grands angles et aux petits angles : tout de suite après (OH), et 24H, 48H et 72H après la lyse.

Plus spécifiquement,

La figure 1 A représente les résultats obtenus pour le contrôle - pas de réactif de lyse,

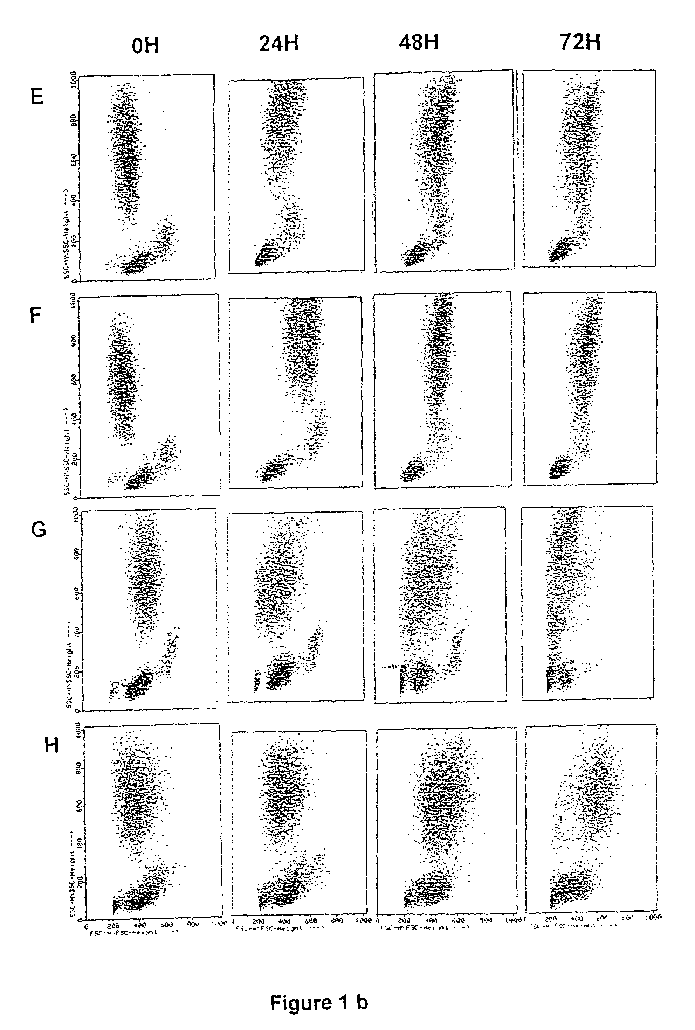

La figure 1 B représente les résultats obtenus pour le réactif de l'exemple 1, en effectuant un lavage avec du tampon phosphate (PBS) 10 mn après la lyse,
La figure 1 C représente les résultats obtenus pour le réactif de l'exemple 3, en effectuant un lavage 10 mn après la lyse,

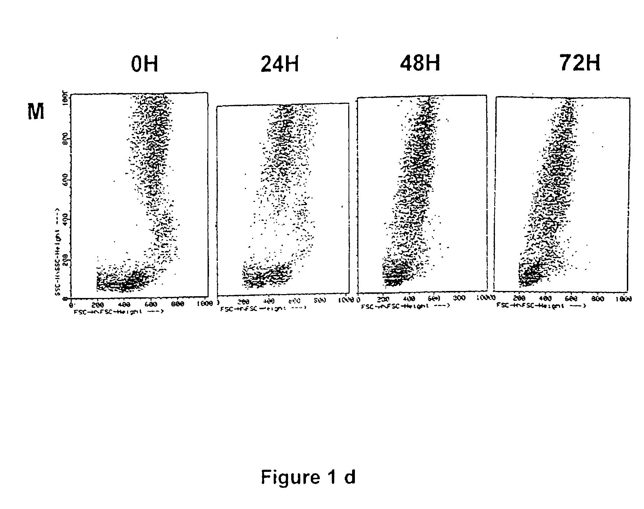

La figure 1 D représente les résultats obtenus pour le réactif de ORTHO DIAGNOSTIC SYSTEMS INC. référence 770521 -agent de lyse = NH$_4$Cl, en effectuant un lavage 10 mn après la lyse,
La figure 1 E représente les résultats obtenus pour le réactif de l'exemple 2,
La figure 1 F représente les résultats obtenus pour le réactif de l'exemple 4,
La figure 1 G représente les résultats obtenus pour le réactif de ORTHO DIAGNOSTICS additionné de 0,3% de HCHO,
La figure 1 H représente les résultats obtenus pour le réactif de l'exemple 1, mais sans lavage,
La figure 1 I représente les résultats obtenus pour le réactif de l'exemple 3, mais sans lavage,
La figure 1 J représente les résultats obtenus pour le réactif de ORTHO DIAGNOSTICS, mais sans lavage,
La figure 1 K représente les résultats obtenus pour le réactif de l'exemple 2, mais sans lavage,
La figure 1 L représente les résultats obtenus pour le réactif de l'exemple 4, mais sans lavage
La figure 1 M représente les résultats obtenus pour le réactif de ORTHO DIAGNOSTICS additionné de 0,3% de HCHO, mais sans lavage.

La figure 2 représente la mise en évidence de la viabilité cellulaire après lyse par l'expansion des cellules T en milieu de culture contenant de l'interleukine 2 et de la phytohaemagglutinine. Le nombre de jours de culture est représenté en abcisse et le nombre de cellules CD3 vivantes est indiqué en ordonnée (en 10$^6$ cellules). Les triangles, les cercles et les carrés représentent respectivement les résultats obtenus avec NH$_4$Cl, l'exemple 1 et le Ficoll.

Les exemples qui suivent illustrent la présente invention.

Préparation de référence

On a utilisé un réactif de lyse à base de NH4Cl (réf. Ortho Diagnostics)

**Exemple 1 : Préparation d'un réactif de lyse**

On a préparé un réactif de lyse répondant à la formule :

0,15M pyrrolidine - HCl
0,04M acide borique
0,005M HEPES
0,0001M EDTA

**Exemple 2 : Préparation d'un réactif de lyse**

On a préparé un réactif de lyse répondant à la formule :

0,15M pyrrolidine - HCl
0,04M acide borique
0,005M HEPES

0,0001M EDTA
0,1M aldéhyde formique
pH 7,0

**Exemple 3 : Préparation d'un réactif de lyse**

On a préparé un réactif de lyse répondant à la formule :

0,15M pipéridine - HCl
0,04M acide borique
0,005M HEPES
0,0001M EDTA
pH : 7,0

**Exemple 4 : Préparation d'un réactif de lyse**

On a préparé un réactif de lyse répondant à la formule :

0,15M pipéridine - HCl
0,04M acide borique
0,005M HEPES
0,0001 M EDTA
0,1M aldéhyde formique

**Etude pharmacologique**

Expérimentation 1 : Mise en évidence de la lyse des érythrocytes

A partir de sang sur EDTA, deux heures après le prélèvement, des échantillons de 100 µl ont été incubés pendant 15 minutes en présence de 20µl des marqueurs monocytaire (CD14-phycoérythrine) et leucocytaire (CD45-FITC), commercialisés par la Société IMMUNOTECH sous la référence IM 1201. Par la suite, les échantillons ont été mélangés avec des préparations de lyse différentes notamment celles décrites ci-dessus, à des températures différentes, soumis à la lyse pendant 10 minutes à température ambiante, puis une partie des échantillons ont été repris en tampon phosphate (PBS) après centrifugation (5mn, 300g). La conservation des échantillons est à 4°C.

Les échantillons ont été analysés sur un cytomètre FACSCAN BECTON & DICKINSON), immédiatement, ou après avoir été lavés avec 3 ml de tampon phosphate et ensuite à des intervalles de 24 heures, durant la conservation.

La figure 1 montre les scattergrammes des préparations de sang lysé analysées à l'aide du programme Simulset (BECTON & DICKINSON). Les pourcentages de lymphocytes, monocytes et granulocytes dans la formule leucocytaire, ont été estimés sur la base du diagramme de diffusion aux grands et petits angles et de la fluorescence CD45/CD14.

Comme contrôle, du sang marqué avec de la CD45-Phycoérytrine (0,1 ml de sang pour 0,02 ml de CD45-Phycoérythrine, dilué 500 fois en PBS sans lyse a été analysé en cytométrie en appliquant un seuil dans le canal de fluorescence FL2 (correspondant à 400 Volts ). Les scattergrammes des événements leucocytaires sont montrés en figure 1A.

En comparant le contrôle sans lyse à la performance du réactif de lyse de l'exemple 1, de l'exemple 2 et du chlorure d'ammonium (fig.1A, B, C et D), on voit la morphologie des monocytes et lymphocytes bien conservés et une légère modification de l'emplacement des polynucléaires vers la droite, surtout dans le cas de $NH_4Cl$.

Pendant la conservation, on observe en plus un déplacement des polynucléaires vers la droite en fonction du temps, indiquant que ce déplacement est associé à une détérioration partielle des polynucléaires. Pendant la conservation de ces échantillons, on voit également un déplacement d'une partie des lymphocytes et des monocytes dans la région des débris, effet qui, apparemment, ne dépend pas de la lyse car il est également observé dans la série de contrôle (Fig. 1A)

Les mêmes phénomènes sont observés dans les séries de conservation après la lyse et sans lavage de la méthode utilisant la préparation de l'exemple 1 et de l'exemple 2 (H,I).

Cela indique que la conservation cellulaire dans les réactifs des exemples 1 et 2 n'est pas différente de la conservation en PBS. En revanche la conservation en $NH_4Cl$ (Fig.1J) montre une dégradation rapide du matériel.

Une très bonne conservation a été obtenue avec les réactifs des exemples 1 et 2, en présence de l'aldéhyde formique après lavage en PBS (Fig. 1E,F) ou sans lavage (Fig. 1K,L).

Malgré le fait que $NH_4Cl$ et l'aldéhyde formique sont incompatibles, on a essayé de faire un mélange en remplaçant le $NH_4Cl$ utilisé par l'aldéhyde formique selon la réaction décrite ci-dessus. Après lyse et lavage, un déplacement des polynucléaires vers la droite a été observé et les lymphocytes sont rapidement détruits et se retrouvent avec les débris (Fig.1G). Apparemment, il reste un peu d'aldéhyde formique dans le mélange, parce que la dégradation dans le produit sans lavage (Fig.1M) est moins importante qu'avec le $NH_4Cl$ sans aldéhyde formique (Fig. 1J).

Sans lavage, le produit de la réaction de $NH_4Cl$ et de l'aldéhyde formique est apparemment toxique pour les cellules car, dans la lyse avec lavage, une dégradation importante avec déplacement des granulocytes vers la gauche et vers le bas s'est produite pendant la conservation (Fig. 1 J).

Expérimentation 2 : Mise en évidence de la viabilité cellulaire après lyse.

Une estimation de la viabilité cellulaire a été obtenue par culture en présence de milieu RPMI 10% sérum de veau foetal et en présence de phytohaemag-

glutinine 10 μg/ml et d'Interleukine 2 (SIGMA, St-Louis, Missouri, 20 unités/ml) des populations leucocytaires ou lymphocytaires après lyse ou séparation. Une population leucocytaire préparée par la méthode de l'exemple 1 a été comparée avec une préparation lymphocytaire préparée selon Boyum (Scandinavian Journal of Clinical and Laboratory investigation 1967-1968. Suppl. 94-101.305.293) sur Ficoll (Histopaque® SIGMA, St-Louis, Missouri) cette dernière étant considérée comme méthode de référence pour la préparation lymphocytaire sans perte de viabilité.

Egalement incluse est une préparation de leucocytes selon la méthode au $NH_4Cl$ (ORTHO DIAGNOSTIC SYSTEMS Co., Raritan New Jersey).

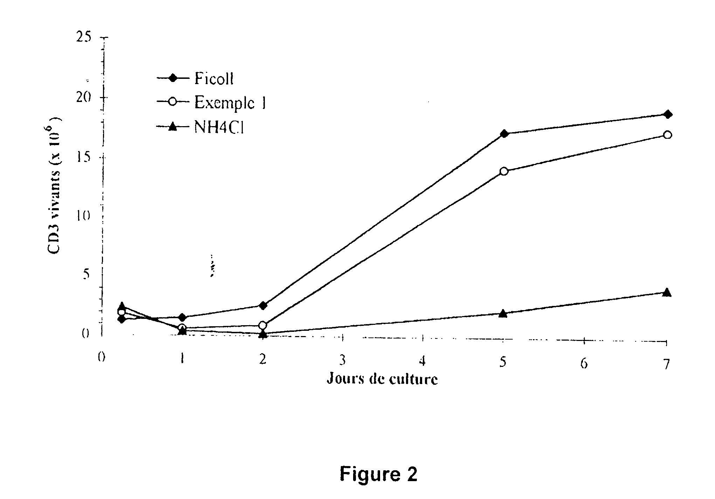

Dans la figure 2, la viabilité a été mise en évidence et estimée à partir du nombre dans la culture de cellules T (CD3+), non nécrotique ou apoptotique selon un marquage avec de l'annexine 5 (WO-A-95/27903). La figure 2 montre la prolifération de cellules T dans le temps sous l'influence de phytohaemagglutinine et Interleukine 2.

En conclusion :

La viabilité après lyse selon l'exemple 1 est substantiellement la même que celle après séparation Histopaque®, tandis que la viabilité après lyse au Chlorure d'Ammonium est fortement réduite.

## Revendications

1. Un réactif de lyse utilisable pour l'analyse cytométrique des leucocytes comprenant de 0,01 à 0,2M d'un agent de lyse qui est un hétérocycle azoté et une quantité efficace d'un composé acide pour conférer audit réactif un pH compris entre 4 et 9.

2. Un réactif de lyse selon la revendication 1, **caractérisé en ce que** l'hétérocycle azoté est utilisé à un pH compris entre 5 et 8.

3. Un réactif de lyse selon l'une des revendications 1 et 2, **caractérisé en ce que** l'hétérocycle azoté utilisé est monocyclique.

4. Un réactif de lyse selon l'une des revendications 1 à 3, **caractérisé en ce que** l'hétérocycle azoté utilisé est saturé.

5. Un réactif de lyse selon l'une des revendications 1 à 4, **caractérisé en ce que** l'hétérocycle azoté utilisé comprend de 3 à 8 atomes de carbone.

6. Un réactif de lyse selon l'une des revendications 1 à 5, **caractérisé en ce que** l'hétérocycle azoté utilisé est choisi parmi la pyrazolidine, l'imidazolidine et l'imidazoline, la pipérazine, la morpholine , la pipéridine ou la pyrrolidine.

7. Un réactif de lyse selon l'une des revendications 1 à 6, **caractérisé en ce qu'**il renferme une quantité efficace d'un agent anticoagulant.

8. Un réactif de lyse selon l'une des revendications 1 à 7, **caractérisé en ce que** le composé utilisé pour conférer le pH désiré comporte le chlorure comme anion.

9. Un réactif de lyse selon la revendication 8, **caractérisé en ce qu'**il comprend, en dehors de l'anion utilisé pour conférer le pH désiré, un contre-ion qui est un carbonate, hydrogénocarbonate ou un borate.

10. Un réactif de lyse selon l'une des revendications 1 à 9, **caractérisé en ce qu'**il comprend un aldéhyde aliphatique.

11. Un réactif de lyse selon l'une des revendications 1 à 10, **caractérisé en ce qu'**il comprend une quantité efficace d'un agent tampon.

12. Un réactif de lyse selon l'une des revendications 1 à 11, **caractérisé en ce qu'**il comprend un mélange de environ 0,15M de chlorhydrate de pyrrolidine, 0,04M d'acide borique, 0,0001M d'ETDA et 0,005M d'HEPES à un pH d'environ 7,0.

13. Une méthode de lyse des érythrocytes dans laquelle on soumet un échantillon de sang total traité avec un agent anti-coagulant à l'action d'un réactif de lyse utilisable pour l'analyse cytométrique des leucocytes, selon l'une des revendications 1 à 12, **caractérisée en ce que** l'agent de lyse est un hétérocycle azoté utilisé à un pH compris entre 4 et 9.

14. Une méthode de lyse des érythrocytes selon la revendication 13, **caractérisée en ce que** le réactif de lyse comprend un mélange de environ 0,15M de chlorhydrate de pyrrolidine, 0,04M d'acide borique, 0,0001M d'ETDA et 0,005M d'HEPES à un pH d'environ 7,0.

## Claims

1. A lysis reagent that can be used for the cytometric analysis of leucocytes, comprising from 0.01 to 0.2 M of a lysis agent which is a nitrogenous heterocyclic compound and an effective quantity of an acid compound to give the said reagent a pH of between 4 and 9.

2. A lysis reagent according to Claim 1, **characterised in that** the nitrogenous heterocyclic compound is used at a pH of between 5 and 8.

3. A lysis reagent according to one of Claims 1 and 2, **characterised in that** the nitrogenous heterocyclic compound that is used is monocyclic.

4. A lysis reagent according to one of Claims 1 to 3, **characterised in that** the nitrogenous heterocyclic compound that is used is saturated.

5. A lysis reagent according to one of Claims 1 to 4, **characterised in that** the nitrogenous heterocyclic compound that is used comprises 3 to 8 carbon atoms.

6. A lysis reagent according to one of Claims 1 to 5, **characterised in that** the nitrogenous heterocyclic compound that is used is chosen from pyrazolidine, imidazolidine and imidazoline, piperazine, morpholine, piperidine or pyrrolidine.

7. A lysis reagent according to one of Claims 1 to 6, **characterised in that** it contains an effective quantity of an anticoagulant agent.

8. A lysis reagent according to one of Claims 1 to 7, **characterised in that** the compound used to give the desired pH comprises chloride as the anion.

9. A lysis reagent according to Claim 8, **characterised in that** it comprises, in addition to the anion that is used to give the desired pH, a counter-ion which is a carbonate, a hydrogen carbonate or a borate.

10. A lysis reagent according to one of Claims 1 to 9, **characterised in that** it comprises an aliphatic aldehyde.

11. A lysis reagent according to one of Claims 1 to 10, **characterised in that** it comprises an effective quantity of a buffer agent.

12. A lysis reagent according to one of Claims 1 to 11, **characterised in that** it comprises a mixture of roughly 0.15M pyrrolidine hydrochloride, 0.04M boric acid, 0.001M EDTA and 0.005M HEPES at a pH of roughly 7.0.

13. A method for the lysis of erythrocytes in which a sample of total blood treated with an anticoagulant agent is subjected to the action of a lysis reagent which can be used for the cytometric analysis of leucocytes according to one of Claims 1 to 12, **characterised in that** the lysis agent is a nitrogenous heterocyclic compound used at a pH of between 4 and 9.

14. A method for the lysis of erythrocytes according to Claim 13, **characterised in that** the lysis reagent comprises a mixture of roughly 0.15Ms pyrrolidine hydrochloride, 0.04M boric acid, 0.0001M EDTA and 0.005M HEPES at a pH of roughly 7.0.

**Patentansprüche**

1. Lysereagens zur Verwendung für die zytometrische Analyse von Leukozyten, umfassend 0,01 bis 0,2 M eines Lysemittels in Form eines Stickstoff-Heterozyklus und eine wirksame Menge einer sauren Verbindung zur Einstellung des Reagens auf einen pH-Wert zwischen 4 und 9.

2. Lysereagens nach Anspruch 1, **dadurch gekennzeichnet, dass** der Stickstoff-Heterozyklus mit einem pH-Wert zwischen 5 und 8 verwendet wird.

3. Lysereagens nach einem der Ansprüche 1 und 2, **dadurch gekennzeichnet, dass** der verwendete Stickstoff-Heterozyklus monocyclisch ist.

4. Lysereagens nach einem der Ansprüche 1 bis 3, **dadurch gekennzeichnet, dass** der verwendete Stickstoff-Heterozyklus gesättigt ist.

5. Lysereagens nach einem der Ansprüche 1 bis 3, **dadurch gekennzeichnet, dass** der verwendete Stickstoff-Heterozyklus 3 bis 8 Kohlenstoffatome aufweist.

6. Lysereagens nach einem der Ansprüche 1 bis 5, **dadurch gekennzeichnet, dass** der verwendete Stickstoff-Heterozyklus ausgewählt ist aus Pyrazolidin, Imidazolidin und Imidazolin, Piperazin, Morpholin, Piperidin oder Pyrrolidin.

7. Lysereagens nach einem der Ansprüche 1 bis 6, **dadurch gekennzeichnet, dass** es eine wirksame Menge eines Antikoagulans enthält.

8. Lysereagens nach einem der Ansprüche 1 bis 7, **dadurch gekennzeichnet, dass** die zur Einstellung des gewünschten pH-Werts verwendete Verbindung das Chlorid als Anion enthält.

9. Lysereagens nach Anspruch 8, **dadurch gekennzeichnet, dass** es abgesehen von dem zur Einstellung des gewünschten pH-Werts verwendeten Anion ein Gegenanion, nämlich ein Carbonat, Hydrogencarbonat oder Borat, enthält.

10. Lysereagens nach einem der Ansprüche 1 bis 9, **dadurch gekennzeichnet, dass** es einen aliphatischen Aldehyd enthält.

**11.** Lysereagens nach einem der Ansprüche 1 bis 10, **dadurch gekennzeichnet, dass** es eine wirksame Menge eines Puffermittels enthält.

**12.** Lysereagens nach einem der Ansprüche 1 bis 11, **dadurch gekennzeichnet, dass** es eine Mischung von etwa 0,15 M Pyrrolidinhydrochlorid, 0,04 M Borsäure, 0,0001 M ETDA und 0,005 M HEPES bei einem pH-Wert von etwa 7,0 enthält.

**13.** Verfahren zur Lyse von Erythrozyten, bei welchem eine mit einem Antikoagulans behandelte Vollblutprobe der Wirkung eines für die zytometrische Analyse von Leukozyten verwendbaren Lysereagens nach einem der Ansprüche 1 bis 12 unterzogen wird, **dadurch gekennzeichnet, dass** das Lysemittel ein Stickstoff-Heterozyklus ist, der bei einem pH-Wert zwischen 4 und 9 verwendet wird.

**14.** Verfahren zur Lyse von Erythrozyten nach Anspruch 13, **dadurch gekennzeichnet, dass** das Lysereagens eine Mischung von etwa 0,15 M Pyrrolidinhydrochlorid, 0,04 M Borsäure, 0,0001 M ETDA und 0,005 M HEPES bei einem pH-Wert von etwa 7,0 enthält.

Figure 1 a

Figure 1 b

Figure 1 c

Figure 1 d

Figure 2